# EUROPÄISCHE PATENTSCHRIFT

(11) **EP 3 797 832 B1**
(45) Veröffentlichungstag und Bekanntmachung des Hinweises auf die Patenterteilung: **05.03.2025**
(21) Anmeldenummer: 20198335.0
(22) Anmeldetag: 25.09.2020
(51) Int. Cl.: A61N 5/10

(54) **APPLIKATOR UND APPLIKATORSYSTEM ZUR ANWENDUNG MIT EINEM STRAHLENTHERAPIEGERÄT**
APPLICATOR AND APPLICATOR SYSTEM FOR APPLICATION WITH A RADIATION THERAPY DEVICE
APPLICATEUR ET SYSTÈME D'APPLICATEUR POUR L'UTILISATION À L'AIDE D'UN APPAREIL DE RADIOTHÉRAPIE

(30) Priorität: 27.09.2019 DE 102019126127
(43) Veröffentlichungstag der Anmeldung: 31.03.2021
(73) Patentinhaber: Carl Zeiss Meditec AG, 07745 Jena (DE)
(72) Erfinder: Weigand, Frank, 89522 Heidenheim (DE)
(74) Vertreter: Diehl & Partner

(56) Entgegenhaltungen:
- EP-A2- 2 335 777
- EP-B1- 2 335 777
- DE-A1- 102012 002 466
- DE-B4- 112013 005 144
- US-A1- 2014 206 925
- VARIAN: "Varian Brachytherapy Applicators and Accessories", 1 January 2011 (2011-01-01), XP055339041, Retrieved from the Internet <URL:https://www.varian.com/sites/default/files/resource_attachments/Brachytherapy_Applicators_Accessories_Catalogue_0_0.pdf> [retrieved on 20170125]

## Beschreibung

Die vorliegende Erfindung liegt in dem Bereich der medizinischen Strahlentherapie und betrifft einen Applikator für ein medizinisches Strahlentherapiegerät, ein Applikatorsystem für ein medizinisches Strahlentherapiegerät und ein Verfahren zur Anwendung eines Applikators bzw. eines Applikatorsystems.

Es existieren medizinische Strahlentherapiegeräte zur lokalen Bestrahlung eines zu therapierenden Körpers mit Strahlung, insbesondere Röntgenstrahlung. Im Bereich der Therapie unter Verwendung von Röntgenstrahlung existieren Therapien, bei denen Röntgenstrahlung im Inneren eines menschlichen oder tierischen Körpers in der Nähe von zu therapierendem Gewebe erzeugt werden soll.

Ein beispielhaftes Strahlentherapiegerät umfasst ein Partikelstrahlsystem, welches einen hochenergetischen Partikelstrahl erzeugen kann. Der Partikelstrahl wird durch ein einige Zentimeter langes Rohr des Strahlentherapiegerätes auf ein Röntgen-Material gerichtet, welches am Ende des Rohres angeordnet ist. Durch Wechselwirkung des Partikelstrahls mit dem Röntgen-Material erzeugt dieses Röntgenstrahlung, welche zur Therapierung von Gewebe vorgesehen ist.

Damit die von dem Röntgen-Material an der Spitze des Rohres erzeugte Röntgenstrahlung im Inneren eines Körpers angewendet werden kann, wird das Rohr in den Körper eingeführt. Hierzu ist das Rohr von einem auf das Strahlentherapiegerät aufsetzbaren Applikator umgeben, welcher einerseits eine sterile Barriere darstellt und andererseits das Rohr des Strahlentherapiegeräts schützt.

Solche Applikatoren müssen aus einem für medizinische Anwendungen zugelassenen Material hergestellt sein (beispielsweise Ultem^{®}). Herkömmlicherweise wird ein Applikator einstückig ausgebildet, beispielsweise indem die finale Form des Applikators aus einem Rohling ausgefräst wird. Das von dem Rohling abgefräste Material kann in der Regel nicht wiederverwendet werden. Dementsprechend ist die Herstellung eines Applikators aufwändig, da eine hochpräzise maschinelle Bearbeitung erforderlich ist, und teuer, da ein großer Anteil des Rohlings zu Ausschuss wird.

In der Regel werden zur Therapierung mittels Röntgenstrahlung mehrere verschiedene Applikatoren unterschiedlicher Größe und Form benötigt. Die Anschaffung eines geeigneten Satzes von Applikatoren ist daher teuer, insbesondere wenn die Applikatoren einstückig ausgebildet sind.

Dementsprechend besteht ein Bedarf nach einem einfacher und günstiger herzustellenden Satz von Applikatoren. Dementsprechend ist es eine Aufgabe der vorliegenden Erfindung, einen Satz von Applikatoren bereitzustellen, welcher einfacher und kostengünstiger hergestellt und flexibler genutzt werden kann.

Die Veröffentlichungen US 2014/0206925 A1; "Brachytherapy-Applicators and Accessories" vom 1. Januar 2011, abgerufen am 25. Januar 2017 von https://www.varian.com/sites/default/files/resource_attachments/Brachytherapy_Ap plicators_Accessories_Catalogue_0_0.pdf; EP 2 335 777 A2; DE 10 2012 002 466 A1; und DE 11 2013 005 144 B4 gehören zum relevanten Stand der Technik und betreffen Applikatoren, Vorrichtungen und Systeme aus dem Bereich der Brachytherapie.

Sie alle lehren keine erfindungsgemäße Ausgestaltung des Applikatorkopfes gemäß Anspruch 1.

Gelöst wird die Aufgabe durch einen Applikator gemäß Anspruch 1.

Ferner wird die Aufgabe gelöst durch ein Applikatorsystem, welches mehrere verschiedene solcher Applikatoren umfasst, wobei der Applikatorrumpf aller Applikatoren derselbe ist. Die Applikatoren des Applikatorsystems können unterschiedliche Größen und/oder Formen aufweisen, wenn die jeweiligen Applikatorköpfe an dem Applikatorrumpf montiert sind.

Lediglich beschrieben wird ein nicht zur Erfindung gehörendes Verfahren, welches umfasst: Bereitstellen eines solchen Applikators, wobei der Applikatorkopf an dem Applikatorrumpf montiert ist; nach dem Bereitstellen, schadloses Demontieren des Applikatorkopfes von dem Applikatorrumpf; und nach dem Demontieren, Montieren des Applikatorkopfes oder eines anderen Applikatorkopfes an dem Applikatorrumpf. Der erfindungsgemäße Applikator zeichnet sich dadurch aus, dass der Applikatorkopf schadlos an dem Applikatorrumpf montierbar ist und von diesem schadlos demontierbar ist. Daher kann ein und derselbe Applikatorrumpf mit verschiedenen Applikatorköpfen verwendet werden. Das heißt, verschiedene Applikatorköpfe können an demselben Applikatorrumpf schadlos montiert und von diesem schadlos demontiert werden. Ein Satz von verschiedenen Applikatoren, welcher hierin auch als Applikatorsystem bezeichnet wird, kann daher einfach durch einen einzigen Applikatorrumpf und verschiedene Applikatorköpfe bereitgestellt werden, da die verschiedenen Applikatorköpfe an ein und demselben Applikatorrumpf schadlos montiert und von diesem schadlos demontiert werden können.

Eine schadlose Montage und schadlose Demontage bedeutet beispielsweise, dass der Applikatorkopf und der Applikatorrumpf im montierten und nicht montierten Zustand jeweils dieselbe körperliche Form aufweisen. Das bedeutet beispielsweise, dass weder die Form des Applikatorkopfes, noch die Form des Applikatorrumpfes durch die Montage und Demontage verändert werden.

Eine schadlose Montage und schadlose Demontage kann zudem oder alternativ bedeuten, dass der Applikatorkopf und der Applikatorrumpf ohne zusätzliche Gegenstände (beispielsweise Klebstoff) aneinander montiert und demontiert werden können.

Beispiele für eine mechanische Verbindung zwischen dem Applikatorkopf und dem Applikatorrumpf, die schadlos geschlossen (schadlose Montage) und gelöst (schadlose Demontage) werden können, sind Steckverbindungen, Schraubverbindungen, kraftschlüssige Verbindung, formschlüssige Verbindungen und dergleichen. Beispiele für mechanische Verbindungen, die nicht schadlos hergestellt und gelöst werden können, sind stoffliche Verbindungen, wie beispielsweise Klebeverbindungen.

Der Applikator kann aus einem für medizinische Zwecke zugelassenen Material gebildet sein. Ein solches Material ist beispielsweise Ultem^{®}. Die einzelnen Komponenten eines Applikators können aus dem gleichen Material gebildet sein. Insbesondere können der Applikatorkopf und der Applikatorrumpf aus demselben Material gebildet sein. Insbesondere können Applikatorkopfelemente des Applikatorkopfes und der Applikatorrumpf aus demselben Material gebildet seien. Der Applikatorkopf kann mehrere Applikatorkopfelemente umfassen, welche unterschiedliches Absorptionsverhalten bezüglich der von dem Strahlentherapiegerät eingesetzten Strahlung haben.

Ausführungsformen der Erfindung werden nachfolgend anhand von Figuren näher erläutert. Hierbei zeigt:
- Figur 1A: eine schematische Darstellung eines Querschnitts eines Strahlentherapiegeräts und eines beispielhaften Applikators in einem demontierten Zustand,
- Figur 1B: eine schematische Darstellung eines Querschnitts des in Figur 1A gezeigten Applikators in einem montierten Zustand, wobei das Strahlentherapiegerät in den Applikator eingeführt ist,
- Figur 2A: eine schematische Darstellung eines Querschnitts eines weiteren beispielhaften Applikators in einem demontierten Zustand,
- Figur 2B: eine schematische Darstellung eines Querschnitts des in Figur 2A gezeigten Applikators in einem montierten Zustand,
- Figur 3A: eine schematische Darstellung eines Querschnitts eines weiteren beispielhaften Applikators in einem demontierten Zustand,
- Figur 3B: eine schematische Darstellung eines Querschnitts des in Figur 3A gezeigten Applikators in einem montierten Zustand,
- Figur 4A: eine schematische Darstellung eines Querschnitts eines weiteren beispielhaften Applikators in einem demontierten Zustand,
- Figur 4B: eine schematische Darstellung eines Querschnitts von Applikatorkopfelementen des in Figur 4A gezeigten Applikators in einem demontierten Zustand, wobei der Querschnitt der Figur 4B senkrecht zu dem Querschnitt der Figuren 4A und 4C orientiert ist,
- Figur 4C: eine schematische Darstellung eines Querschnitts des in Figur 4A gezeigten Applikators in einem montierten Zustand, und
- Figur 5: ein beispielhaftes Verfahren zur Anwendung eines Applikators.

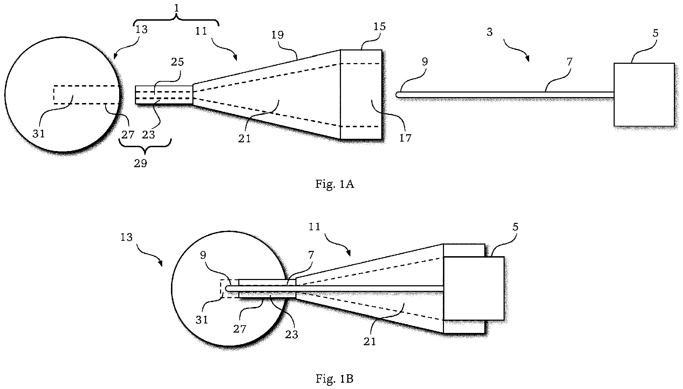

Nachfolgend wird eine erste Ausführungsform eines Applikators 1 für ein medizinisches Strahlentherapiegerät 3 mit Bezug zu den Figuren 1A und 1B beschrieben. Figur 1A zeigt eine schematische Darstellung eines Querschnitts durch den Applikator 1 und das Strahlentherapiegerät 3, wobei der Applikator 1 in einem demontierten Zustand dargestellt ist, d. h. in einem Zustand, in welchem ein Applikatorkopf 13 des Applikators 1 nicht an einem Applikatorrumpf 11 des Applikators 1 montiert ist, und das Strahlentherapiegerät 3 nicht in den Applikator 1 eingeführt ist. Figur 1B zeigt eine schematische Darstellung eines Querschnitts durch den Applikator 1 und das Strahlentherapiegerät 3, wobei der Applikator 1 in einem montierten Zustand dargestellt ist, d. h. in einem Zustand, in welchem der Applikatorkopf 13 an dem Applikatorrumpf 11 montiert ist, und das Strahlentherapiegerät 3 in den Applikator 1 eingeführt ist.

Das Strahlentherapiegerät 3 umfasst einen Hauptkörper 5 mit einem sich von dem Hauptkörper 5 weg erstreckenden Rohr 7. An einem von dem Hauptkörper 5 entfernten Ende 9 des Rohres 7 kann (im Inneren des Rohres 7) ein Röntgen-Material angeordnet seien, welches dazu geeignet ist, Röntgenstrahlung zu emittieren.

Das Strahlentherapiegerät 3 ist dazu konfiguriert, die Röntgenstrahlung mittels des Röntgen-Materials zu erzeugen. Hierzu kann das Strahlentherapiegerät 3 ein in den Figuren nicht gezeigtes, im Inneren des Hauptkörpers 5 angeordnetes Partikelstrahlsystem umfassen, welches einen Partikelstrahl im Inneren des Rohres 7 auf das Röntgen-Material richtet, welches unter Wechselwirkung mit dem Partikelstrahl die Röntgenstrahlung emittiert.

Der Applikator 1 umfasst einen Applikatorrumpf 11 und einen Applikatorkopf 13. Der Applikatorrumpf 11 und der Applikatorkopf 13 sind separate Teile, die jeweils einstückig ausgebildet sind.

Der Applikatorrumpf 11 umfasst einen Adapterabschnitt 15, welcher dazu konfiguriert ist, mit dem Strahlentherapiegerät 3 verbunden zu werden. Insbesondere ist der Adapterabschnitt 15 dazu konfiguriert, mit dem Hauptkörper 5 des Strahlentherapiegeräts 3 verbunden zu werden. In dem in Figur 1A gezeigten Beispiel ist der Adapterabschnitt 15 ein im Wesentlichen röhrenförmiger Abschnitt mit einem Innenraum 17, in welchem der Hauptkörper 5 des Strahlentherapiegeräts 3 wenigstens teilweise angeordnet werden kann.

Der Applikatorrumpf 11 umfasst ferner einen Mittelabschnitt 19, der sich an den Adapterabschnitt 15 anschließt. Der Mittelabschnitt 19 weist eine konisch zulaufende röhrenförmige Form auf. Der Mittelabschnitt 19 umgibt einen Innenraum 21, welcher mit dem Innenraum 17 des Adapterabschnitts 15 zusammenhängt.

Der Applikatorrumpf 11 umfasst ferner einen Verbindungsabschnitt 23. Der Verbindungsabschnitt 23 weist eine im Wesentlichen röhrenförmige Form auf. Ein von dem Verbindungsabschnitt 23 umschlossener Innenraum 25 schließt sich an den Innenraum 21 des Mittelabschnitts 19 an. Der Verbindungsabschnitt 23 dient zum Verbinden (Montieren) des Applikatorrumpfes 11 mit dem Applikatorkopf 13.

Der Applikatorkopf 13 weist in dem Beispiel der Figur 1A eine im Wesentlichen kugelförmige Außenform auf. Die Außenform des Applikatorkopfes 13 kann jedoch auch eine beliebige andere gewünschte Außenform haben, beispielsweise eine ellipsoide Form. Die Form des Applikatorkopfes wird in Abhängigkeit des zu therapierenden Körpers gewählt. Bei einer Therapieanwendung (Bestrahlung) wird der Applikator 1 in den zu therapierenden Körper eingeführt, so dass wenigstens ein Teil des Applikatorkopfes 13 in den zu therapierenden Körper eingeführt ist. Die Oberfläche des Applikatorkopfes 13 stützt dabei das zu therapierende Gewebe und dient zudem der Erzeugung eines gewünschten Strahlungsprofils.

Der Applikatorkopf 13 umfasst einen Verbindungsabschnitt 27. In dem in Figur 1A gezeigten Beispiel ist der Verbindungsabschnitt 27 des Applikatorkopfes 13 eine zylinderförmige Ausnehmung im Inneren des Applikatorkopfes 13, die sich bis zur Oberfläche erstreckt.

Der Applikatorkopf 13 umfasst ferner einen Innenraum 31, in welchem das Ende 9 des Strahlentherapiegeräts 3 angeordnet ist, wenn der Applikatorkopf 13 an dem Applikatorrumpf 11 montiert ist. In dem in den Figuren 1A und 1B gezeigten Beispiel ist die Ausnehmung, die den Verbindungsabschnitt 27 bereitstellt, ein Teil des Innenraums 31.

Der Verbindungsabschnitt 23 des Applikatorrumpfes 11 und der Verbindungsabschnitt 27 des Applikatorkopfes 13 bilden zusammen eine erste Montagevorrichtung 29, welche dazu konfiguriert ist, eine schadlos lösbare mechanische Verbindung zwischen dem Applikatorkopf 13 und dem Applikatorrumpf 11 bereitzustellen. In dem in Figur 1A gezeigten Beispiel stellt die erste Montagevorrichtung 29 eine Steckverbindung bereit, die durch die Verbindungsabschnitte 23 und 27 gebildet werden kann. Das bedeutet, dass der Applikatorkopf 13 an dem Applikatorrumpf 11 montiert werden kann (mit diesem mechanisch verbunden werden kann), indem der Verbindungsabschnitt 23 in den Verbindungsabschnitt 27 eingeführt wird, wobei durch Reibschluss eine kraftschlüssige Verbindung zwischen dem Applikatorkopf 13 und dem Applikatorrumpf 11 gebildet wird. Diese mechanische Verbindung kann schadlos gelöst werden, indem der Applikatorkopf 13 von dem Applikatorrumpf 11 abgezogen wird.

In Figur 1B ist der Applikatorkopf 13 an dem Applikatorrumpf 11 montiert. Die Montage erfolgt mittels der oben beschriebenen Steckverbindung durch die Verbindungsabschnitte 23 und 27. Der Applikator 1 ist damit in einem montierten Zustand.

Zudem ist das Strahlentherapiegerät 3 in den montierten Applikator 1 eingeführt. Der Hauptkörper 5 des Strahlentherapiegeräts 3 befindet sich dabei teilweise im Innenraum 17 des Adapterabschnitts 15 des Applikatorrumpfes 11. Ferner durchläuft das Rohr 7 des Strahlentherapiegeräts 3 die Innenräume 21 und 25 des Applikators 1 und tritt aus einem vorderen Ende des Verbindungsabschnitts 23 aus dem Applikator 1 heraus. Das Ende 9 des Strahlentherapiegeräts 3 befindet sich in dem Innenraum 31. Mithin befindet sich das Ende 9 des Strahlentherapiegeräts 3 annähernd in der Mitte des Applikatorkopfes 13.

Die erste Montagevorrichtung 29 ist so ausgebildet, dass sie eine schadlos lösbare mechanische Verbindung zwischen dem Applikatorkopf 13 und dem Applikatorrumpf 11 bereitstellen kann. Wie in dem Beispiel in Figur 1A gezeigt, sind die Verbindungsabschnitte 23 und 27, die die erste Montagevorrichtung 29 bilden, als Abschnitte des Applikatorkopfes 13 und des Applikatorrumpfes 11 ausgebildet. Daher sind der Applikatorkopf 13 und der Applikatorrumpf 11 so ausgebildet, dass der Applikatorkopf 13 an dem Applikatorrumpf 11 schadlos montierbar und von diesem schadlos demontierbar ist.

Diese Konfiguration des Applikators 1 erlaubt die Verwendung mehrerer verschiedener Applikatorköpfe mit ein und demselben Applikatorrumpf 11. Ein Satz von Applikatoren, auch bezeichnet als Applikatorsystem, kann daher durch einen Applikatorrumpf und mehrere (verschiedene) Applikatorköpfe bereitgestellt werden, wobei jeder der Applikatorköpfe einzeln an dem Applikatorrumpf schadlos montierbar und von diesem schadlos demontierbar ist.

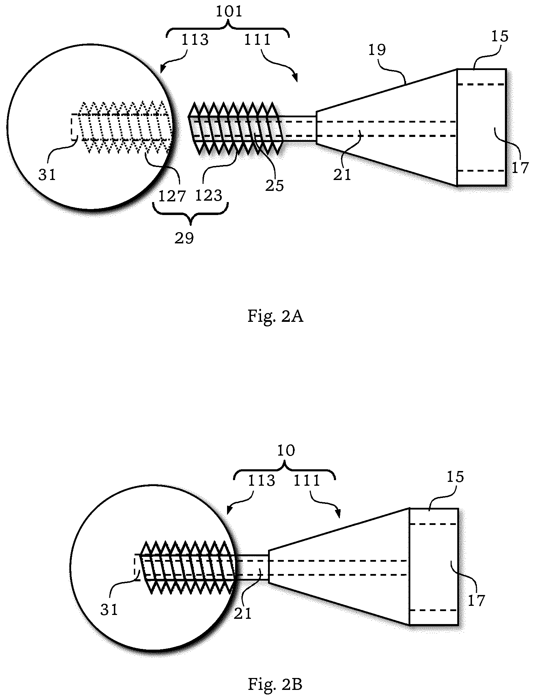

Eine zweite Ausführungsform eines Applikators 101 wird nachfolgend mit Bezug zu den Figuren 2A und 2B beschrieben, wobei Figur 2A eine schematische Darstellung eines Querschnitts durch den Applikator 101 in einem demontierten Zustand zeigt, während die Figur 2B eine schematische Darstellung eines Querschnitts durch den Applikator 101 in einem montierten Zustand zeigt.

Der Applikator 101 umfasst einen Applikatorrumpf 111 und einen Applikatorkopf 113. Der Applikatorrumpf 111 unterscheidet sich von dem in Figur 1 gezeigten Applikatorrumpf 11 im Wesentlichen nur durch die konkrete Ausgestaltung des Verbindungsabschnitts. Dementsprechend werden lediglich die Unterschiede zu dem in Figur 1A gezeigten Applikatorrumpf 11 beschrieben. Für die mit gleichen Bezugszeichen versehenen Komponenten gilt die Beschreibung zu Figur 1A. Der Applikatorrumpf 111 umfasst einen Verbindungsabschnitt 123, welcher in dem in Figur 2A gezeigten Beispiel durch ein Außengewinde gebildet ist.

Ferner umfasst der Applikator 101 den Applikatorkopf 113, dessen Außenform dem Applikatorkopf 13 in Figur 1A entspricht. Der Applikatorkopf 113 umfasst einen Verbindungsabschnitt 127, welcher in dem in Figur 2A gezeigten Beispiel ein Innengewinde ist, welches zu dem Verbindungsabschnitt 123 (Außengewinde) des Applikatorrumpfes 111 passt. Der Verbindungsabschnitt 127 liegt innerhalb der im Wesentlichen kugelförmigen Oberfläche des Applikatorkopfes 113. Die Verbindungsabschnitte 123 und 127 bilden zusammen die erste Montagevorrichtung 29.

Der Applikatorkopf 113 kann an dem Applikatorrumpf 111 schadlos montiert werden, indem die Verbindungsabschnitte 123 und 127 miteinander verschraubt werden, so wie dies in Figur 2B dargestellt ist. Der Applikatorkopf 113 ist von dem Applikatorrumpf 111 schadlos demontierbar, indem der Applikatorkopf 113 von dem Applikatorrumpf 111 abgeschraubt wird, so wie dies in Figur 2A gezeigt ist. In dem in den Figuren 2A und 2B gezeigten Beispiel ist die erste Montagevorrichtung 29 eine Schraubverbindung.

Mit dem in den Figuren 2A und 2B gezeigten Applikator 101 können die gleichen Wirkungen erzielt werde wie mit dem in den Figuren 1A und 1B gezeigten Applikator 1.

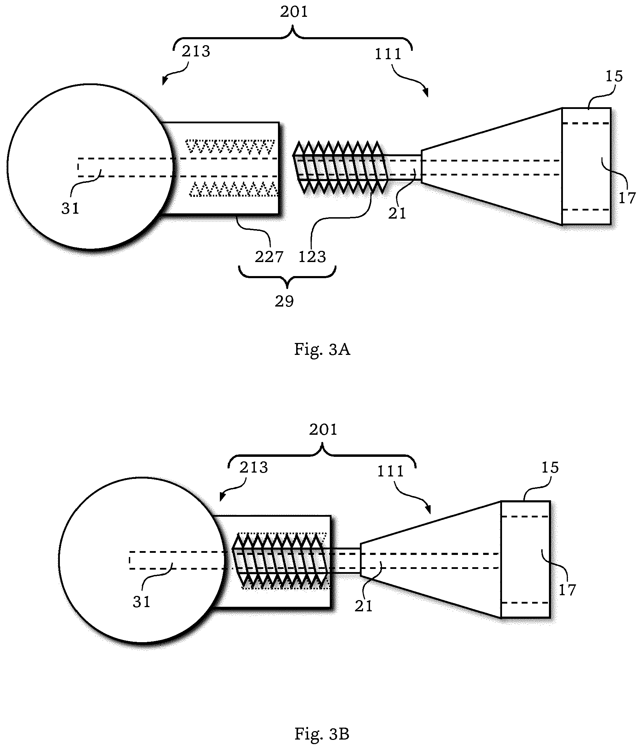

Eine dritte Ausführungsform eines Applikators 201 wird nachfolgend mit Bezug zu den Figuren 3A und 3B beschrieben, wobei Figur 3A eine schematische Darstellung eines Querschnitts durch den Applikator 201 in einem demontierten Zustand zeigt, während die Figur 3B eine schematische Darstellung eines Querschnitts durch den Applikator 201 in einem montierten Zustand zeigt.

Der Applikator 201 umfasst den Applikatorrumpf 111 und einen Applikatorkopf 213. Der Applikatorrumpf 111 entspricht dem in Zusammenhang mit dem Applikator 101 beschriebenen Applikatorrumpf.

Die Außenform des Applikatorkopfes 213 entspricht der des Applikatorkopfes 13 in Figur 1A. Der Applikatorkopf 213 umfasst einen Verbindungsabschnitt 227, welcher in dem in Figur 3A gezeigten Beispiel ein Innengewinde ist, welches zu dem Verbindungsabschnitt 123 (Außengewinde) des Applikatorrumpfes 111 passt. Der Verbindungsabschnitt 227 liegt außerhalb der im Wesentlichen kugelförmigen Oberfläche des Applikatorkopfes 213. Die Verbindungsabschnitte 123 und 227 bilden zusammen die erste Montagevorrichtung 29.

Der Applikatorkopf 213 kann an dem Applikatorrumpf 111 schadlos montiert werden, indem die Verbindungsabschnitte 123 und 227 miteinander verschraubt werden, so wie dies in Figur 3B dargestellt ist. Der Applikatorkopf 213 ist von dem Applikatorrumpf 111 schadlos demontierbar, indem der Applikatorkopf 213 von dem Applikatorrumpf 111 abgeschraubt wird, so wie dies in Figur 3A gezeigt ist. In dem in den Figuren 3A und 3B gezeigten Beispiel ist die erste Montagevorrichtung 29 eine Schraubverbindung.

Mit dem in den Figuren 3A und 3B gezeigten Applikator 201 können die gleichen Wirkungen erzielt werden wie mit den in den Figuren 1A, 1B, 2A und 2B gezeigten Applikatoren 1 und 101.

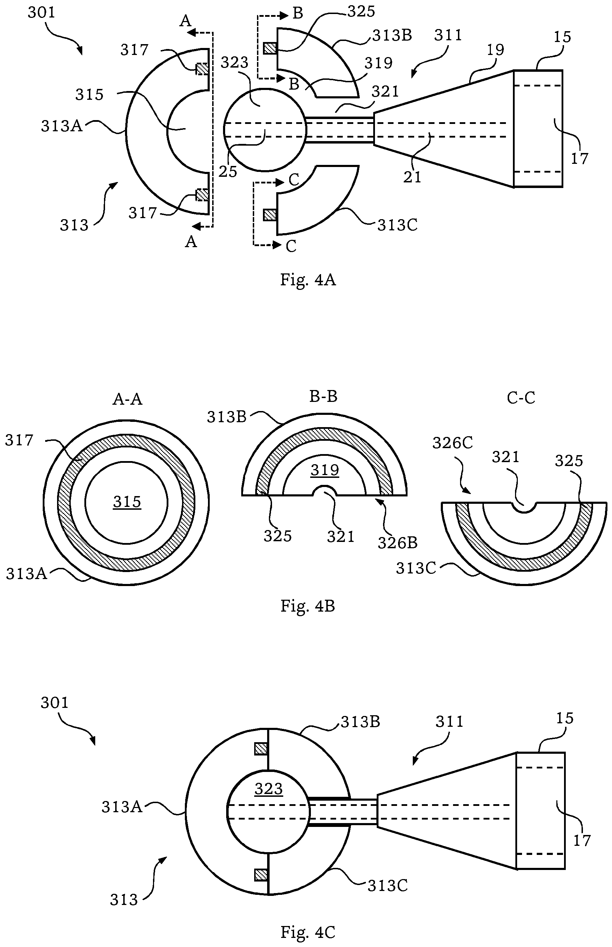

Eine vierte Ausführungsform eines Applikators 301 wird nachfolgend mit Bezug zu den Figuren 4A bis 4C beschrieben.

Figur 4A zeigt eine schematische Darstellung eines Querschnitts durch den Applikator 301 in einem demontierten Zustand. Figur 4B zeigt eine schematische Darstellung eines Querschnitts von Applikatorkopfelementen des in Figur 4A gezeigten Applikators 301 in einem demontierten Zustand, wobei der Querschnitt der Figur 4B senkrecht zu dem Querschnitt der Figuren 4A und 4C orientiert ist. Figur 4C zeigt eine schematische Darstellung eines Querschnitts durch den in Figur 4A gezeigten Applikator 301 in einem montierten Zustand.

Der Applikator 301 umfasst einen Applikatorrumpf 311 und einen Applikatorkopf 313.

Der Applikatorrumpf 311 unterscheidet sich von dem in Figur 1A gezeigten Applikatorrumpf 11 im Wesentlichen nur dadurch, dass der Verbindungsabschnitt 323 des Applikatorrumpfes 311 eine im Wesentlichen kugelförmige Außenform aufweist.

Der Applikatorkopf 313 umfasst mehrere Applikatorkopfelemente. In dem in den Figuren 4A bis 4C gezeigten Beispiel umfasst der Applikatorkopf 313 drei separate Applikatorkopfelemente 313A, 313B und 313C. Figur 4B zeigt eine Darstellung des Applikatorkopfelements 313A im Querschnitt A-A, der senkrecht zu der Zeichenebene der Figur 4A orientiert ist, entlang der Pfeilrichtung, d. h. in Figur 4A nach links. Figur 4B zeigt ferner eine Darstellung des Applikatorkopfelements 313B im Querschnitt B-B, der senkrecht zu der Zeichenebene der Figur 4A orientiert ist, entlang der Pfeilrichtung, d. h. in Figur 4A nach rechts. Figur 4B zeigt ferner eine Darstellung des Applikatorkopfelements 313C im Querschnitt C-C, der senkrecht zu der Zeichenebene der Figur 4A orientiert ist, entlang der Pfeilrichtung, d. h. in Figur 4A nach rechts.

Das Applikatorkopfelement 313A hat eine im Wesentlichen halbkugelförmige Außenform mit einer halbkugelförmigen Ausnehmung 315, welche der Größe des Verbindungsabschnitts 323 des Applikatorrumpfes 311 entspricht. Das Applikatorkopfelement 313A weist ferner ein Innengewinde 317 auf.

Das Applikatorkopfelement 313B hat eine im Wesentlichen kugelsegmentförmige Außenform, und weist zudem zwei Ausnehmungen 319 und 321 auf. Die Ausnehmung 319 ist eine kugelsegmentförmige Ausnehmung, deren Form und Größe auf die Größe des Verbindungsabschnitts 323 des Applikatorrumpfes 311 angepasst ist. Die Ausnehmung 321 ist eine zylinderförmige Ausnehmung, die auf die Form und Größe des Mittelabschnitts 19 des Applikatorrumpfelements 311 angepasst ist. Das Applikatorkopfelement 313B umfasst ferner ein Außengewinde 325. Das Außengewinde 325 ist auf das Innengewinde 317 angepasst.

Das Applikatorkopfelement 313C entspricht im Wesentlichen dem Applikatorkopfelement 313B, wobei das Außengewinde 325 des Applikatorkopfelements 313C und das Außengewinde 325 des Applikatorkopfelements 313B so aufeinander abgestimmt sind, dass sich aus diesen ein funktional zusammenwirkendes Außengewinde ergibt, das in das Innengewinde 317 des Applikatorkopfelements eingeschraubt werden kann, wenn eine Seite 326B des Applikatorkopfelements 313B (siehe Figur 4B) und eine Seite 326C des Applikatorkopfelements 313C aneinander gelegt sind

Die Applikatorkopfelemente 313A, 313B und 313C sind so ausgebildet, dass sie aneinander schadlos montierbar und voneinander schadlos demontierbar sind. In dem in den Figuren 4A bis 4C gezeigten Beispiel stellen das Innengewinde 317 und das aus den Außengewinden 325 der Applikatorkopfelemente 313B und 313C funktional zusammenwirkende Außengewinde eine zweite Montagevorrichtung bereit, welche dazu konfiguriert ist, eine schadlos lösbare mechanische Verbindung zwischen den Applikatorkopfelementen 313A bis 313C bereitzustellen. Beispielsweise werden die Applikatorkopfelemente 313A bis 313C aneinander schadlos montiert, indem die Applikatorkopfelemente 313B und 313C an den Seiten 326B und 326C aneinander gelegt werden, um das funktional zusammenwirkende Außengewinde zu bilden, welches anschließend in das an dem Applikatorkopfelement 313A bereitgestellte Innengewinde 317 eingeschraubt wird. Eine solche Situation, in welchem die Applikatorkopfelemente 313A bis 313C aneinander montiert sind, ist in Figur 4C gezeigt.

Der Applikatorkopf 313 kann an dem Applikatorrumpf 311 wie folgt schadlos montiert werden: Das Applikatorkopfelement 313A wird auf den Verbindungsabschnitt 323 des Applikatorrumpfes 311 gesetzt, wobei der Verbindungsabschnitt 323 in die Ausnehmung 315 des Applikatorkopfelements 313A eingeführt wird. Das Applikatorkopfelement 313B und das Applikatorkopfelement 313C werden an den Seiten 326B und 326C aneinander gelegt, wobei der Mittelabschnitt 19 des Applikatorrumpfes 311 die Ausnehmung 321 durchläuft. Hierbei sind das Applikatorkopfelement 313A und die aneinander gelegten Applikatorkopfelemente 313B und 313C voneinander entlang einer Längsrichtung des Applikators (in Figur 4A in der Richtung von links nach rechts) beabstandet, damit die Außengewinde 325 beim Aneinanderlegen der Applikatorkopfelemente 313B und 313C nicht mit dem Applikatorkopfelement 313A kollidieren. In diesem Zustand kann der Verbindungsabschnitt 323 bereits teilweise in die Ausnehmungen 319 eingeführt sein. Anschließend werden das Applikatorkopfelement 313A und die aneinander gelegten Applikatorkopfelemente 313B und 313C entlang der Längsrichtung aneinander geschoben. Anschließend werden die aneinander gelegten Applikatorkopfelemente 313B und 313C mit dem Applikatorkopfelement 313A verschraubt, indem das die aneinander gelegten Applikatorkopfelemente 313B und 313C relativ zu dem Applikatorkopfelement 313A um die Längsachse gedreht werden, wodurch das Innengewinde 317 und das funktional zusammenwirkende Außengewinde ineinandergreifen.

Die Applikatorkopfelemente 313A bis 313C können voneinander schadlos demontiert werden, indem die Schraubverbindung gelöst wird, so wie dies in Figur 4A gezeigt ist.

Die Figuren 4A bis 4C zeigen ein Beispiel für die zweite Montagevorrichtung in Form einer Schraubverbindung. Die zweite Montagevorrichtung kann jedoch auch durch eine andere Art der Verbindung bereitgestellt werden, insbesondere in Form einer Steckverbindung, einer kraftschlüssigen Verbindung oder einer formschlüssigen Verbindung.

In dem in den Figuren 4A bis 4C gezeigten Beispiel sind die Ausnehmung 315 des Applikatorkopfelements 313A und die Ausnehmung 319 der Applikatorkopfelemente 313B und 313C so ausgebildet, dass der Verbindungsabschnitt 323 des Applikatorrumpfes 311 von den Applikatorkopfelementen 313A bis 313C wenigstens teilweise, insbesondere im Wesentlichen vollständig und bündig umschlossen sein kann. Hierdurch entspricht die Abstrahlcharakteristik des Applikatorkopfes 313 im Wesentlichen der Abstrahlcharakteristik eines einstückig ausgebildeten Applikatorkopfes mit der gleichen Außenform.

Mit anderen Worten können die Applikatorkopfelemente 313A bis 313C um den Verbindungsabschnitt 323 angeordnet werden und anschließend (mittels der zweiten Montagevorrichtung) miteinander verbunden werden, so wie dies in Figur 4C dargestellt ist. Im aneinander montierten Zustand bilden die Applikatorkopfelemente 313A bis 313C eine kugelförmige Schale mit passenden Ausnehmungen für den Verbindungsabschnitt 323 und den Mittelabschnitt 19 des Applikatorrumpfes 311. Wenn die Applikatorkopfelemente 313A bis 313C auf diese Weise aneinander montiert sind und dabei um den Verbindungsabschnitt 323 angeordnet sind, so wie dies in Figur 4C gezeigt ist, entsteht hierdurch eine formschlüssige Verbindung zwischen dem aus den Applikatorkopfelementen 313A bis 313C gebildeten Applikatorkopf 313 und dem Applikatorrumpf 311. Diese formschlüssige Verbindung ist ein Beispiel für die erste Montagevorrichtung 29. Auf diese Weise wird im Wege der durch die zweite Montagevorrichtung bereitgestellten mechanischen Verbindung zwischen den Applikatorkopfelementen 313A bis 313C zudem eine schadlos lösbare mechanische Verbindung zwischen dem Applikatorkopf 313 und dem Applikatorrumpf 311 gebildet.

In dem Beispiel der Figuren 4A bis 4C weist der Verbindungsabschnitt 323 eine im Wesentlichen kugelförmige Außenform auf. Andere Außenformen sind möglich, insbesondere eine im Wesentlichen ellipsoide Außenform.

In dem Beispiel der Figuren 4A bis 4C weist der Applikatorkopf 313, welcher durch die aneinander montierten Applikatorkopfelemente 313A bis 313C gebildet wird, eine im Wesentlichen kugelförmige Außenform auf. Andere Außenformen sind möglich, insbesondere eine im Wesentlichen ellipsoide Außenform.

Die individuellen Formen der Komponenten der vorangehend beschriebenen Applikatoren sind nicht auf die in den Ausführungsformen gezeigten Formen beschränkt.

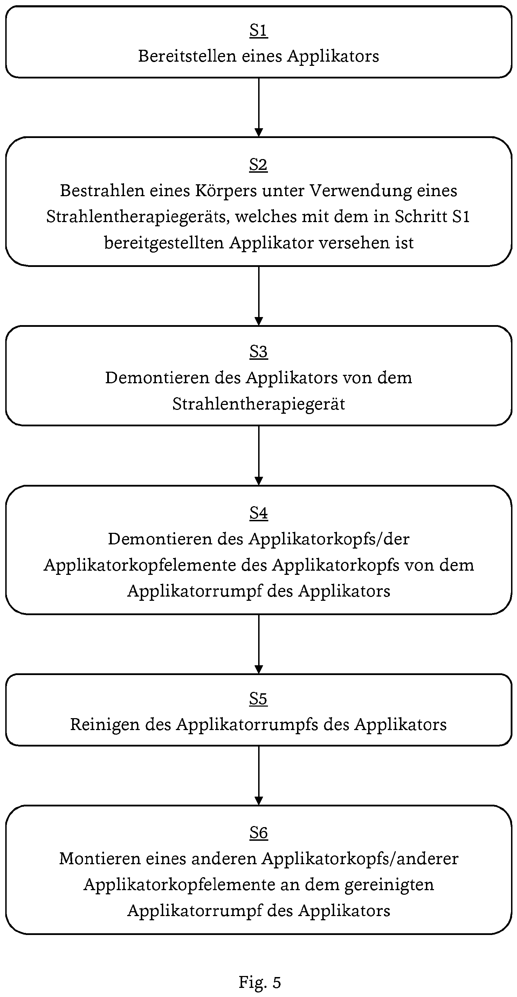

Figur 5 zeigt ein beispielhaftes Verfahren zur Anwendung eines der hierin beschriebenen Applikatoren.

In einem ersten Schritt S1 wird ein Applikator bereitgestellt. Der Applikator kann beispielsweise einer der vorangehend beschriebenen Applikatoren 1, 101, 201 oder 301 sein. Vorliegend wird zur Beschreibung stellvertretend auf die Applikatoren1 und 301 Bezug genommen.

In einem darauffolgenden Schritt S2 wird ein zu behandelnder Körper unter Verwendung des in Schritt S1 bereitgestellten Applikators 1 und eines Strahlentherapiegeräts 3 bestrahlt. Wie beispielhaft in Figur 1B dargestellt, wird hierzu der Applikatorkopf 13 an dem Applikatorrumpf 11 montiert und das Strahlentherapiegerät 3 wird über den Adapterabschnitt 15 in den Applikator 1 eingeführt, so dass die in Figur 1B skizzierte Situation erzielt wird. Das Strahlentherapiegerät 3, an welchem der Applikator 1 auf diese Weise angebracht ist, wird nun in den zu therapierenden Körper eingeführt, wobei das Applikatorkopfelement 13 wenigstens teilweise in den zu therapierenden Körper eingeführt wird. Anschließend wird durch das Strahlentherapiegerät 3 eine Strahlung, beispielsweise Röntgenstrahlung, am Ende 9 erzeugt und über den Applikatorkopf 13 an den zu therapierenden Körper abgegeben.

Nach der Bestrahlung in Schritt S2 kann der Applikator von dem Strahlentherapiegerät in Schritt S3 demontiert werden, so wie dies in Figur 1A gezeigt ist.

In einem nachfolgenden Schritt S4 wird der Applikatorkopf 13 bzw. werden die Applikatorkopfelemente 313A bis 313C von dem Applikatorrumpf 11 bzw. 311 schadlos demontiert. Im Falle der Figuren 1A und 1B wird der Applikatorkopf 13 durch Lösen der Steckverbindung zwischen dem Applikatorkopf 13 und dem Applikatorrumpf 11 von dem Applikatorrumpf 11 demontiert. Im Falle der Figuren 4A bis 4C werden die Applikatorkopfelemente 313A bis 313C von dem Applikatorrumpf 311 schadlos demontiert, indem zunächst die Schraubverbindung zwischen den Applikatorkopfelementen 313A bis 313C gelöst und anschließend die formschlüssige Verbindung zwischen den Applikatorkopfelementen 313A bis 313C und dem Applikatorrumpf 311 gelöst wird.

In einem nachfolgenden Schritt S5 kann der Applikatorrumpf 11 gereinigt werden. Somit kann der Applikatorrumpf 11 wiederverwendet werden. Der Applikatorrumpf 11 bildet den größten Teil des Applikators 1 und es ist daher besonders vorteilhaft, den Applikatorrumpf 11 mehrfach zu benutzen. Hingegen können der Applikatorkopf bzw. die Applikatorkopfelemente deutlich kleiner und daher günstiger als der Applikatorrumpf sein. Der Applikatorkopf bzw. die Applikatorkopfelemente können daher als Einmalprodukte verwendet werden, d. h. Produkte, die nur einmalig zum Einsatz kommen.

Zur Vorbereitung einer weiteren Bestrahlung kann gemäß Schritt S6 ein Applikator bereitgestellt werden, indem ein anderer Applikatorkopf bzw. andere Applikatorkopfelemente an dem gereinigten Applikatorrumpf montiert wird bzw. werden. Alternativ können auch der Applikatorkopf bzw. die Applikatorkopfelemente gereinigt und in Schritt S6 wieder an dem gereinigten Applikatorrumpf montiert werden. Auf diese Weise können sämtliche Teile eines Applikators wiederholt verwendet werden.

## Patentansprüche

1. Applikator (301) für ein medizinisches Strahlentherapiegerät (3) zur Therapierung mittels Strahlung, wobei der Applikator (301) umfasst:
einen Applikatorkopf (313) und einen Applikatorrumpf (311), wobei der Applikatorkopf und der Applikatorrumpf so ausgebildet sind, dass der Applikatorkopf an dem Applikatorrumpf schadlos montierbar und von diesem schadlos demontierbar ist;
wobei der Applikatorkopf (313) mindestens zwei separate
Applikatorkopfelemente (313A, 313B, 313C) umfasst, welche so ausgebildet sind, dass sie aneinander schadlos montierbar und voneinander schadlos demontierbar sind.

2. Applikator (301) nach Anspruch 1, wobei der Applikatorkopf (313) und der Applikatorrumpf (311) eine erste Montagevorrichtung (29) umfassen, welche dazu konfiguriert ist, eine schadlos lösbare mechanische Verbindung zwischen dem Applikatorkopf und dem Applikatorrumpf bereitzustellen.

3. Applikator (301) nach Anspruch 2, wobei die erste Montagevorrichtung (29) eine Schraubverbindung, eine Steckverbindung, eine formschlüssige Verbindung, eine kraftschlüssige Verbindung oder dergleichen zwischen dem Applikatorkopf und dem Applikatorrumpf bereitstellt.

4. Applikator (301) nach einem der Ansprüche 1 bis 3, wobei die Applikatorkopfelemente (313A, 313B, 313C) eine zweite Montagevorrichtung (317, 325) umfassen, welche dazu konfiguriert ist, eine schadlos lösbare mechanische Verbindung zwischen den Applikatorkopfelementen bereitzustellen.

5. Applikator (301) nach einem der Ansprüche 1 bis 4, wobei die Applikatorkopfelemente (313A, 313B, 313C) und der Applikatorrumpf (311) so ausgebildet sind, dass eine schadlos lösbare mechanische Verbindung zwischen dem Applikatorrumpf und den Applikatorkopfelementen hergestellt ist, wenn die Applikatorkopfelemente aneinander montiert sind.

6. Applikator (301) nach einem der Ansprüche 1 bis 5,
wobei ein Abschnitt (323) des Applikatorrumpfes von dem Applikatorkopf im Wesentlichen umschlossen ist, wenn der Applikatorkopf (313) an dem Applikatorrumpf (311) montiert ist; und
wobei der Abschnitt (323) eine im Wesentlichen kugelförmige Außenform oder eine im Wesentlichen ellipsoide Außenform aufweist.

7. Applikator (301) nach einem der Ansprüche 1 bis 6, wobei der Applikatorkopf (313) eine im Wesentlichen kugelförmige Außenform oder eine im Wesentlichen ellipsoide Außenform aufweist, wenn der Applikatorkopf an dem Applikatorrumpf (311) montiert ist.

8. Applikator (301) nach einem der Ansprüche 1 bis 7, wobei der Applikatorrumpf (311) und der Applikatorkopf (313) aus dem gleichen Material gebildet sind.

9. Applikator nach einem der Ansprüche 1 bis 8, wobei die Applikatorkopfelemente ein unterschiedliches Absorptionsverhalten bezüglich der Strahlung haben.

10. Applikator nach einem der Ansprüche 1 bis 9, wobei der Applikatorrumpf (311) einstückig ausgebildet ist.

11. Applikator nach einem der Ansprüche 1 bis 10,
wobei der Applikatorrumpf (311) einen Adapterabschnitt (15) umfasst, welcher dazu konfiguriert ist, mit dem Strahlentherapiegerät (3) verbunden zu werden;
wobei der Applikatorrumpf (311) ferner einen Mittelabschnitt (19) umfasst, der sich an den Adapterabschnitt (15) anschließt und einen Innenraum (21) umgibt, welcher mit einem Innenraum (17) des Adapterabschnitts (15) zusammenhängt; wobei der Applikatorrumpf (311) ferner einen Verbindungsabschnitt (323) umfasst, welcher einen Innenraum (25) umschließt, der sich an den Innenraum (21) des Mittelabschnitts (19) anschließt;
wobei ein Rohr (7) des Strahlentherapiegeräts (3) den Innenraum (21) des Mittelabschnitts (19) und den Innenraum (25) des Verbindungsabschnitts (323) durchläuft, wenn das Strahlentherapiegerät (3) in den Applikator (311) eingeführt ist.

12. Applikator nach Anspruch 11, wobei der Adapterabschnitt (15) ein im Wesentlichen röhrenförmiger Abschnitt mit dem Innenraum (17) ist, in welchem ein Hauptkörper (5) des Strahlentherapiegeräts (3) wenigstens teilweise anordenbar ist.

13. Applikatorsystem, welches mehrere verschiedene Applikatoren gemäß einem der Ansprüche 1 bis 12 umfasst, wobei der Applikatorrumpf aller Applikatoren derselbe ist.

14. Applikatorsystem gemäß Anspruch 13, wobei die Applikatoren unterschiedliche Größen und/oder Formen aufweisen, wenn die jeweiligen Applikatorköpfe an dem Applikatorrumpf montiert sind.

## Claims

1. Applicator (301) for a medical radiotherapy device (3) for performing therapy by means of radiation, the applicator (301) comprising:
an applicator head (313) and an applicator body (311), wherein the applicator head and the applicator body are designed such that the applicator head can be assembled on, and disassembled from, the applicator body, in each case without damage;
wherein the applicator head (313) comprises at least two separate applicator head elements (313A, 313B, 313C), which are designed such that they can be assembled on, and disassembled from, one another, in each case without damage.

2. Applicator (301) according to Claim 1, wherein the applicator head (313) and the applicator body (311) comprise a first assembling mechanism (29), which is configured to provide a mechanical connection between the applicator head and the applicator body, which can be released without damage.

3. Applicator (301) according to Claim 2, wherein the first assembling mechanism (29) provides a screw connection, a plug connection, an interlocking connection, a force-fit connection or the like between the applicator head and the applicator body.

4. Applicator (301) according to any of Claims 1 to 3, wherein the applicator head elements (313A, 313B, 313C) comprise a second assembling mechanism (317, 325), which is configured to provide a mechanical connection between the applicator head elements, which can be released without damage.

5. Applicator (301) according to any of Claims 1 to 4, wherein the applicator head elements (313A, 313B, 313C) and the applicator body (311) are designed such that a mechanical connection between the applicator body and the applicator head elements, which can be released without damage, is established when the applicator head elements are assembled on one another.

6. Applicator (301) according to any of Claims 1 to 5,
wherein a section (323) of the applicator body is substantially surrounded by the applicator head when the applicator head (313) is assembled on the applicator body (311); and
wherein the section (323) has a substantially spherical external shape or a substantially ellipsoid external shape.

7. Applicator (301) according to any of Claims 1 to 6, wherein the applicator head (313) has a substantially spherical external shape or a substantially ellipsoid external shape when the applicator head is assembled on the applicator body (311).

8. Applicator (301) according to any of Claims 1 to 7, wherein the applicator body (311) and the applicator head (313) are made of the same material.

9. Applicator according to any of Claims 1 to 8, wherein the applicator head elements have different absorption characteristics in respect of the radiation.

10. Applicator according to any of Claims 1 to 9, wherein the applicator body (311) is formed in one piece.

11. Applicator according to any of Claims 1 to 10,
wherein the applicator body (311) comprises an adapter section (15) which is configured to be connected to the radiotherapy device (3);
wherein the applicator body (311) further comprises a central section (19) which adjoins the adapter section (15) and surrounds an interior space (21) that is associated with an interior space (17) of the adapter section (15);
wherein the applicator body (311) further comprises a connecting section (323) which encloses an interior space (25) that adjoins the interior space (21) of the central section (19);
wherein a tube (7) of the radiotherapy device (3) passes through the interior space (21) of the central section (19) and the interior space (25) of the connecting section (323) when the radiotherapy device (3) is inserted into the applicator (311).

12. Applicator according to Claim 11, wherein the adapter section (15) is a substantially tubular section with the interior space (17), in which a main body (5) of the radiotherapy device (3) is at least partially arrangeable.

13. Applicator system comprising a plurality of different applicators according to any of Claims 1 to 12, wherein the applicator body of all applicators is the same.

14. Applicator system according to Claim 13, wherein the applicators have different sizes and/or shapes when the respective applicator heads are assembled on the applicator body.

## Revendications

1. Applicateur (301) pour un appareil de radiothérapie médicale (3) pour la thérapie par rayonnement, l'applicateur (301) comprenant :
une tête (313) d'applicateur et un corps (311) d'applicateur, la tête d'applicateur et le corps d'applicateur étant conçus de telle sorte que la tête d'applicateur soit apte à être montée sans dommage sur le corps d'applicateur et soit apte à être démontée sans dommage de celui-ci ;
dans lequel la tête (313) d'applicateur comprend au moins deux éléments (313A, 313B, 313C) de tête d'applicateur séparés, qui sont formés de manière à pouvoir être mutuellement montés sans dommage et à pouvoir être mutuellement démontés sans dommage.

2. Applicateur (301) selon la revendication 1, dans lequel la tête (313) d'applicateur et le corps (311) d'applicateur comprennent un premier dispositif de montage (29) configuré pour assurer une liaison mécanique démontable sans dommage entre la tête d'applicateur et le corps d'applicateur.

3. Applicateur (301) selon la revendication 2, dans lequel le premier dispositif de montage (29) fournit une connexion par vis, une connexion par fiche, une connexion par complémentarité de formes, une connexion par force ou similaire entre la tête d'applicateur et le corps d'applicateur.

4. Applicateur (301) selon l'une des revendications 1 à 3, dans lequel les éléments (313A, 313B, 313C) de tête d'applicateur comprennent un deuxième dispositif de montage (317, 325) configuré pour fournir une connexion mécanique démontable sans dommage entre les éléments de tête d'applicateur.

5. Applicateur (301) selon l'une des revendications 1 à 4, dans lequel les éléments (313A, 313B, 313C) de tête d'applicateur et le corps (311) d'applicateur sont conçus de manière à établir une liaison mécanique démontable sans dommage entre le corps d'applicateur et les éléments de tête d'applicateur lorsque les éléments de tête d'applicateur sont montés ensemble.

6. Applicateur (301) selon l'une des revendications 1 à 5,
dans lequel une partie (323) du corps d'applicateur est sensiblement entourée par la tête d'applicateur lorsque la tête (313) d'applicateur est montée sur le corps (311) d'applicateur ; et
dans lequel la partie (323) a une forme extérieure sensiblement sphérique ou une forme extérieure sensiblement ellipsoïdale.

7. Applicateur (301) selon l'une des revendications 1 à 6, dans lequel la tête (313) d'applicateur présente une forme extérieure sensiblement sphérique ou une forme extérieure sensiblement ellipsoïdale lorsque la tête d'applicateur est montée sur le corps (311) d'applicateur.

8. Applicateur (301) selon l'une des revendications 1 à 7, dans lequel le corps (311) d'applicateur et la tête (313) d'applicateur sont formés dans le même matériau.

9. Applicateur selon l'une des revendications 1 à 8, dans lequel les éléments de la tête d'applicateur ont un comportement d'absorption différent par rapport au rayonnement.

10. Applicateur selon l'une des revendications 1 à 9, dans lequel le corps (311) d'applicateur est formé d'une seule pièce.

11. Applicateur selon l'une des revendications 1 à 10,
dans lequel le corps (311) d'applicateur comprend une partie adaptatrice (15) configurée pour être connectée à l'appareil de radiothérapie (3) ;
dans lequel le corps (311) d'applicateur comprend en outre une partie centrale (19) qui se raccorde à la partie adaptatrice (15) et entoure un espace intérieur (21) qui est contigu à un espace intérieur (17) de la partie adaptatrice (15) ;
le corps (311) d'applicateur comprend en outre une partie de connexion (323) qui entoure un espace intérieur (25) qui se raccorde à l'espace intérieur (21) de la partie centrale (19) ;
un tube (7) de l'appareil de radiothérapie (3) traverse l'espace intérieur (21) de la partie centrale (19) et l'espace intérieur (25) de la partie de connexion (323) lorsque l'appareil de radiothérapie (3) est inséré dans l'applicateur (311).

12. Applicateur selon la revendication 11, dans lequel la partie adaptatrice (15) est une partie sensiblement tubulaire avec l'espace intérieur (17) dans lequel un corps principal (5) de l'appareil de radiothérapie (3) est apte à être agencé au moins partiellement.

13. Système d'applicateurs comprenant plusieurs applicateurs différents selon l'une des revendications 1 à 12, le corps de tous les applicateurs étant le même.

14. Système d'applicateurs selon la revendication 13, dans lequel les applicateurs ont des tailles et/ou des formes différentes lorsque les têtes d'applicateurs respectives sont montées sur le corps de l'applicateur.
